**Europäisches Patentamt**

**European Patent Office**

**Office européen des brevets**

(19)

(11) Publication number : **0 283 125 B1**

## EUROPEAN PATENT SPECIFICATION

(12)

(45) Date of publication of patent specification :
04.03.92 Bulletin 92/10

(51) Int. Cl.⁵ : **A61M 1/00**

(21) Application number : **88301174.4**

(22) Date of filing : **12.02.88**

(54) **Surgical drainage apparatus.**

(30) Priority : **13.02.87 US 14435**

(43) Date of publication of application :
**21.09.88 Bulletin 88/38**

(45) Publication of the grant of the patent :
**04.03.92 Bulletin 92/10**

(84) Designated Contracting States :
**AT BE CH DE ES FR GB GR IT LI LU NL SE**

(56) References cited :
**US-A- 4 036 231**
**US-A- 4 312 351**
**US-A- 4 354 493**
**US-A- 4 439 189**
**US-A- 4 533 353**

(73) Proprietor : **SHERWOOD MEDICAL COMPANY**
**1915 Olive Street**
**St. Louis, Missuri 63103 (US)**

(72) Inventor : **Kerwin, Michael John**
**826, Village Meadow Drive**
**Ballwin Missouri 63011 (US)**

(74) Representative : **Porter, Graham Ronald et al**
**c/o Wyeth Laboratories Huntercombe Lane**
**South**
**Taplow Maidenhead Berkshire SL6 0PH (GB)**

EP 0 283 125 B1

Jouve, 18, rue Saint-Denis, 75001 PARIS

## Description

This invention relates to surgical drainage apparatus and, more particularly, to a drainage device for collecting fluids from the thoracic cavity.

Thoracic drainage devices are normally used to remove gases and air as well as blood or other liquids from the pleural cavity surrounding the lungs. Typically, such drainage is needed after chest surgery, after infliction of a chest wound or in some cases simply to remove excessive amounts of fluid that may accumulate as a result of an illness, such as pneumonia.

Thoracic drainage is carried out either under gravity or by application of a slight suction. One type of suction drainage system that has been long employed is known as a three bottle system in which one bottle is used to collect the body fluids, a second bottle provides a liquid seal to prevent backflow of the fluids to the patient, and a third bottle regulates the level of suction. The bottles are often of glass.

Various devices have been developed to replace the breakable glass bottles used in prior art systems and to combine the functions of two or even three of the bottles into a single unit. For example one such device combines a collection container with a liquid seal chamber. It is desirable that the liquid seal be maintained at all times because if the seal is lost the drainage unit may not function properly and the patient's life maybe placed in danger.
Coughing or gasping can deplete the liquid seal and allow backflow of fluids and solids to the patient; this may make patient breathing more difficult and may also introduce infection.

It is highly desirable to monitor bubbling of gas through the liquid seal since this gives an indication of the strength and condition of the patient's respiratory system. Visibility of the lower part of the liquid seal inlet tube is also very desirable in order to observe the rise and fall of liquids (tidalling) in response to changes in pressure in the pleural cavity. This is a useful indication of the strength & regularity of patient breathing. One problem with prior art devices is that the liquid seal chamber may fill with foam thus obscuring the inlet tube and making it difficult for an observer to monitor bubbling through the liquid in the chamber.

It is also essential to prevent blockage of internal fluid passages by blood clots and the like. Any blockage which prevents free flow of air is likely to rapidly render breathing very difficult and puts the patient at risk. Furthermore, blockage of the inlet tube may result in fluids backing up to the patient's body cavity. Consequently, it is desirable to avoid small openings & passages if the drainage unit is intended for more than a few hours use.

US-A-4354493 discloses a thoracic drainage device having a collection chamber for receiving fluids from a body cavity to be drained, said drainage device comprising a water trap chamber for receiving the fluids, said water trap chamber further containing a predetermined quantity of liquid, inlet means to said water trap chamber and with means to communicate with the body cavity to be drained, said inlet means disposed within said water trap chamber at a point below the level of said initial quantity of liquid, outlet means from said water trap chamber to carry liquid from said water trap chamber to said collection chamber as said liquid reaches a predetermined level in said water trap chamber, receptacle means carried by said drainage device and means to receive a container containing the initial quantity of liquid, channel means providing a flow path for the liquid received in said receptacle means to said water trap chamber and baffle means in said channel means intermediate said receptacle means and said water trap chamber to prevent any return flow of the liquid from the water trap chamber to said receptacle.

US-A-4533353 discloses a dry tape discharge liquid extraction apparatus for a thoracic procedure comprising:
a container having a main body opened at the front side including,
a suction pressure adjusting chamber formed at the upper portion thereof and provided with a nozzle for connection to a suction source,
a liquid seal tube provided with a liquid seal chamber and a nozzle for connection to a human body cavity tube, and
a main storage chamber in communication with a liquid seal chamber of said liquid seal tube, and a front plate secured on the front opened side of the main body,
the container being provided with,
a suction pressure adjusting valve means in communication with said suction pressure adjusting chamber and said main storage chamber,
a high negative pressure adjusting valve means for adjusting excess pressure in the human body cavity,
a valve means for releasing positive and negative pressure in said container, and
a suction pressure stabilizing valve in communication with said suction pressure adjusting chamber and outside air.

One object of the present invention is to provide a thoracic drainage apparatus which has an easily observed inlet tube and liquid seal chamber.

Another object of the invention is to provide a liquid seal chamber which retains liquid even if the apparatus is tipped over. The invention also provides a liquid seal chamber which is not susceptible to blockage by blood clots and like material

The invention is a substantial improvement over known drainage devices, and overcomes many of the disadvantages and shortcomings of devices used for the same or similar purposes.

According to the invention there is provided a chest drainage device having a fluid collection chamber and a closed transparent liquid seal chamber including an overflow outlet in fluid flow connection with said container, characterised thereby that said device includes a removable assembly removably fixed and sealed within said chamber, said removable assembly including an inlet, a transparent inlet tube spaced from a side wall of said chamber and in fluid flow connection with said inlet, said inlet tube forming a lower edge opening therein which is located within said chamber between an overflow opening of said overflow tube and a bottom wall of said chamber, and a baffle wall extending from said inlet tube to said side walls, said baffle wall providing an enclosed space through which, in use, fluid in said inlet tube may be viewed.

Such an arrangement ensures that, even though the seal chamber may fill with foam, the inlet tube remains visible so that bubbling and tidalling can be observed.

Preferably the seal chamber includes a further baffle wall extending from the inlet tube to the inner side of the chamber to define an inner chamber around the overflow tube, the further wall extending below the level of the overflow tube and having one or more apertures above that level.

This additional baffle wall ensures that the inner chamber contains only liquid whereas foam is directed by the additional wall to the space between the inner chamber and the inner side of the liquid seal chamber. As the foam breaks down liquid sinks into the liquid seal and gas escapes through the apertures in the additional wall into the inner chamber from where it passes through the overflow tube to the collection container.

The additional wall maybe cut away at the base to ease the passage of fluid to the inner chamber, the cut away portions are below the level of the overflow tube. The additional wall is 'U' shaped in plan, the inlet tube being at the base of the 'U' and the limbs being cut away adjacent their ends.

The apertures in the additional wall may be shielded from rising foam by a further baffle extending between the inner chamber and the inner side of the seal chamber and extending around one side and below each respective aperture.

In the preferred embodiment the inlet tube and baffle walls are moulded to a removeable cap of the liquid seal chamber. This facilitates assembly of the unit and enables the seal chamber to be dismantled should that prove necessary.

The baffle walls should fit sufficiently closely to the inner side of the seal chamber wall to prevent passage of foam. The removeable cap may be orientated in the seal chamber by location means such as a lug and notch.

The seal chamber may be inserted into an upstanding tubular portion of the collection container.

This again facilitates assembly and the seal chamber may be secured, for example by a threaded ring. In the preferred embodiment the removeable cap is retained in the seal chamber by the same threaded ring. It is important that the seal chamber be reasonably air tight to allow operation under vacuum and in the preferred embodiment an elastomeric ring is provided to seal the seal chamber in the container.

The passages in the liquid seal chamber are preferably larger than the inlet tube diameter to prevent blockage by blood clots and the like. Any matter which passes down the inlet tube from the patient will thus pass through the liquid seal chamber into the collection container.

Other features of the invention will be apparent from the following description of a preferred embodiment shown by way of example only in the accompanying drawings in which:-

Fig. 1 is a front elevation of thoracic drainage unit according to the present invention;

Fig. 2 is a front elevation of the liquid seal chamber and associated baffle assembly of the drainage unit shown in Fig. 1;

Fig. 2A is a plan view of an elastomeric ring seal for the liquid seal chamber;

Fig. 2B is an enlarged cross-section taken on line B-B of Fig. 2A;

Fig. 3 is a front elevation of the inlet tube and baffle assembly

Fig. 4 is a plan view of the assembly of Fig. 3;

Fig. 5 is a side elevation of the inlet tube and baffle assembly of Fig. 3 shown positioned in the liquid seal chamber of Fig. 2 which is shown in cross-section; and

Fig. 6 is an exploded perspective view of the liquid seal chamber.

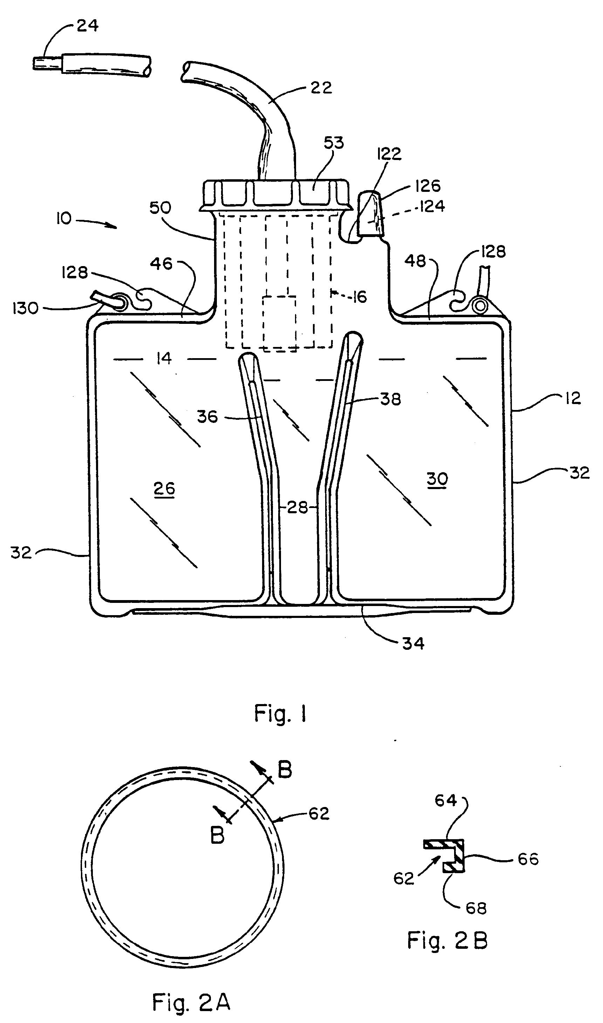

Referring to the drawings Fig. 1 shows a thoracic drainage unit 10 constructed of a relatively rigid transparent plastic and including a container 12 having a three compartment fluid collection chamber 14 and a liquid seal chamber 16 located in an upper portion 50 of the container 12.

An inlet tube 22 opens into the liquid seal chamber and is adapted for connection to a thoracic drainage tube 24.

The collection chamber 14 is divided by partition walls 36,38 into compartments 26,28, and 30. Walls 36 and 38 are preferably formed by folds in the peripheral wall 32, as shown in Fig. 1. A septum connecting the chambers may run down the lateral centreline of the container; this prevents undue flexing.

The partition 36 is somewhat shorter than the partition 38 and both partitions have V-shaped upper edges. In use, body fluids first accumulate in the middle compartment 28; eventually the fluids overflow into compartment 26 and finally the fluid rises sufficiently to overflow into compartment 30 thereby filling the entire container. The container 12 also has upper walls 46 and 48 and an upwardly extending tubular wall 50 with an opening 52 to receive the liquid seal assembly 16. The opening 52 is round and has external threads for cooperation with a closure ring 53. The liquid seal assembly 16 and the baffle assembly 54 positioned therein are assembled as a unit for insertion into the opening 52.

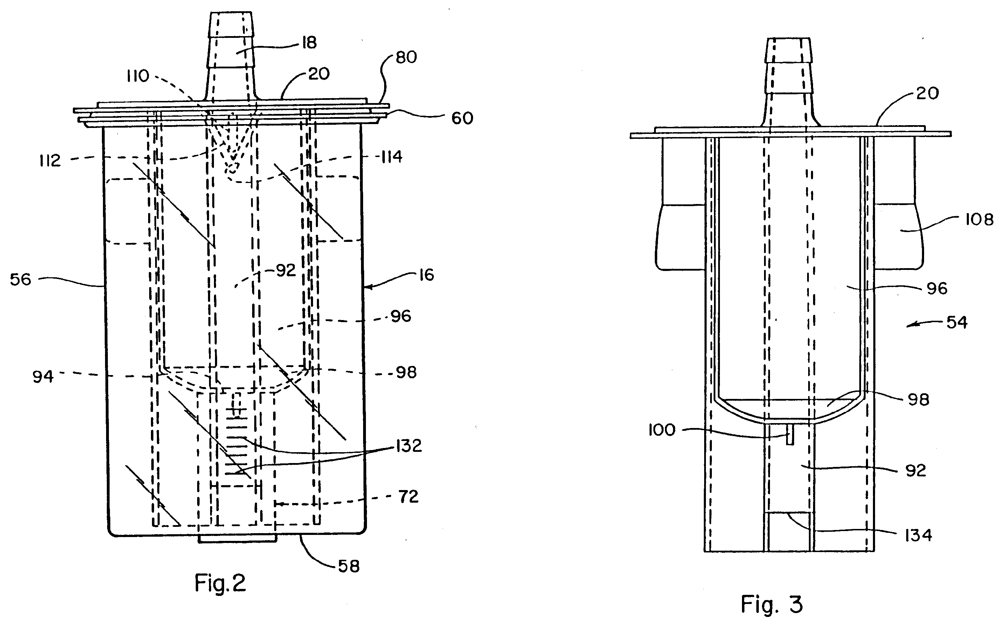

The details of liquid seal chamber and the associated baffle assembly 54 are shown in Figs. 2-6. The liquid seal assembly 16 includes circular wall 56 closed at the bottom by wall 58. The upper edge of the wall 56 has an outwardly extending annular flange 60 which is embraced by a circular seal 62. The seal is formed by connected portions 64, 66 and 68 as shown in figs 2A & 2B. The bottom wall 58 of the assembly 16 has a central opening 70 in which an upstanding overflow tube 72 is positioned; the tube protudes slightly below the wall 58 as shown.

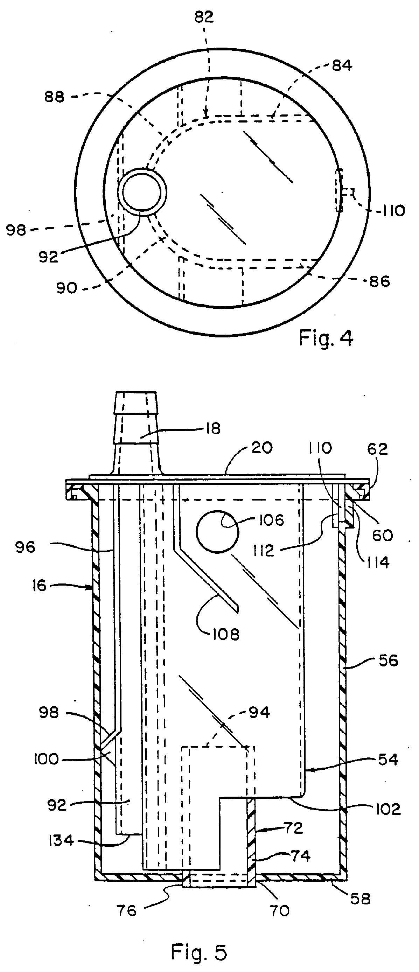

The baffle assembly 54 (fig 3) extends downwardly into the chamber 16 and has an upper closure wall 20 which abuts the seal 62. The assembly 54 includes a depending curved wall 82 comprising parallel wall portions 84 and 86 and curved wall portions 88 and 90 (Fig 4) which extend from opposite sides of a vertical tube 92. The tube 92 extends downwardly from the wall 20 and terminates between the bottom wall 58, and the upper open end of tube 72 as shown.

An upright wall 96 is attached to the outer side of tube 92; the side edges of wall 96 are beveled to make surface contact with the inner face of the chamber wall 56 when the baffle assembly is inserted in the chamber. The lower end of the wall 96 is connected to an outwardly and downwardly sloping wall 98 which also has its edges shaped to make contact with inner surface of the wall 56. As will be described below the walls 96,98 establish a liquid free space so that the tube 92 can be seen through wall 56. The wall 98 is shown braced to the tube 92 by another wall portion 100.

Referring to Fig. 4, it can be seen that the spaced wall portions 84 and 86 also extend to engage the wall 56 so as to form a substantially enclosed space therewithin. The wall portions 84 & 86 are cut away at their lower edge as shown (reference numeral 102). The cut away portions are below the upper open end of tube 72.

Openings 106 are formed in walls 84 and 86 near the upper ends thereof as shown in Fig. 5, and depending V-shaped baffles 108 are mounted on either side of the baffle assembly 54. The baffles 108 are similar and extend away from the respective openings 106 for contact with the wall 56.

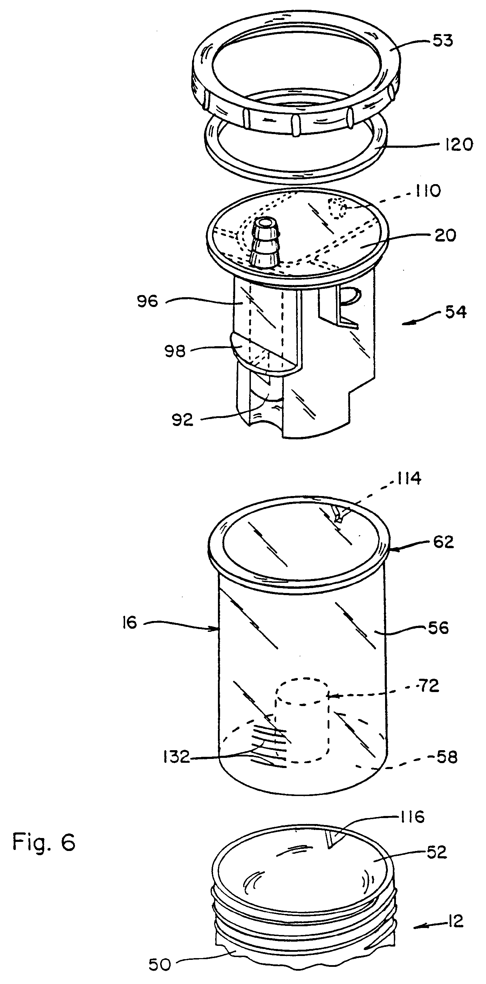

The upper wall 20 of the baffle assembly 54 has a locating lug 110 which cooperates with a notch 112 in the wall of the seal chamber 16. In like manner, the seal chamber 16 has a triangular lug 114 which cooperates with a locating notch 116 (Fig. 6) in the neck portion of the main container 12. The locating lugs and associated notches ensure that the main container 12, seal chamber 16 and baffle assembly 54 can be quickly assembled in the correct orientation.

The upper wall 20 of the baffle assembly 54 has a tubular inlet fitting 18 which is shaped to receive one end of an inlet hose 22, the opposite end of which is connected to a thoracic drainage catheter.

In use, the seal chamber 16 and baffle assembly 54 are inserted into the container neck 50 and retained by the closure ring 53; the seal 62 ensures a good air-tight closure for operation under suction.

The container 12 has a tubular outlet fitting 124 extending upwardly therefrom and which can be closed by a cap 126. A vacuum line (not shown) can be attached to the fitting 124 to assist in drawing fluids from the body cavity. The fitting 124 provides a vent when drainage is under gravity.

The container 12 also has hooks 128 and/or a strap 130 for hanging the device in a convenient location. Alternatively the container 12 can stand on a flat surface below the level of the patient. Stability of a free standing container can be improved by using transverse bracing members which fit into the slots formed by partition walls 36, 38. Such members may be wire or flat plastic supports which extend outwardly from the container.

On the front portion of the liquid seal chamber 16 are a plurality of spaced lines 132 which provide a visual indication of the level of liquid in the seal chamber 16. The level should be maintained high enough to establish the necessary liquid seal taking into account the conditions of use. Liquid from the patient can accumulate in the chamber 16 until the liquid level exceeds the height of tube 72 whereupon it overflows into the central chamber portion 28 of the main container 12.

The lower end of the inlet tube 92 is cut away on the outer side so that a space is formed between the lower front edge 134 and bottom wall 58 of the chamber 16. This facilitates drainage of incoming fluids and permits the inner side of the tube 92 to complete the baffle wall 82.

In a preferred embodiment the outer portion of the tube 92 is cut away by about 9-13mm ($\frac{3}{8}$ to $\frac{1}{2}$ inch) as measured from the bottom wall 58, a distance that is at least as large as the inside diameter of the inlet tube 92. The sloping wall 98 is located somewhat above the open lower tube end 134 to prevent incoming fluids from splashing and making it difficult to observe the operation of the device.

When assembled into the liquid seal chamber 16, the curved wall portions 88 and 90 of the baffle extend all the way to the bottom wall 58 of the chamber 16 while the parallel baffle walls 84 and 86 are cut away to provide fluid communication on opposite sides thereof. The openings 106 communicate with the interior of the baffle and serve mainly as outlets for the gases that may be present. This provides expansion space for the gases which space also communicates through the baffle assembly 54 and through the outlet tube 72 to collection chamber 14.

The baffles 108 are intended to deflect rising substances which may block the openings 106. The outer edges of the baffle assembly 54 are bevelled to make contact with the inner surface of the chamber 16. Generally the outer surfaces of the baffle assembly fit closely with the inner surface of the chamber 16 but do not necessarily provide a fluid tight seal therebetween; seals are not required between all of the adjacent surfaces for the device to operate but it is desirable to substantially reduce or to prevent the passage of foam between the various baffle chambers and to direct liquid to the openings provided in the lower portions of the chamber 16 and gases to the upper openings 106.
The relatively close fit of adjacent walls is sufficient to prevent solid and semi-solid substances or foams moving therebetween.

In use, it has been found desirable to coat the baffle assembly 54 with a medical antifoam solution which prevents undue buildup of foamed liquid at, for example, the gas openings 106. The coating also prevents foam buildup which might deplete the liquid seal. A suitable antifoam solution comprises Dow Corning Medical Grade Antifoam A dispersed in a volatile solvent such as a Freon (Registered Trade Mark) or methylene chloride, a preferred volatile solvent being Freon TF of E.I. Dupont de Nemours and Company. A suitable solution concentration ranges from about 4% to about 10% of the Antifoam A dissolved in from about 90 to about 96% by weight of solvent. The baffle assembly 54 is dipped in this solution until coated up to the lower surface of the cover 20 and the solvent is allowed to flash off, either at ambient conditions or in a low temperature air recirculating oven. The coating of the antifoam agent remaining on the baffle assembly 54 is very effective in preventing the buildup of persistent foam in the liquid seal chamber 16 and this is true even under continuous bubbling of the gases through the liquid seal. As noted above the size and extent of such air bubbles which can be viewed in the subject device affords an easily monitored indication of the patient's respiration conditions. The antifoam agent also acts to prevent premature depletion of the liquid seal due to bubbles generated by drainage of a patient pneumothorax or bloody haemorrhage which can cause extensive bubbling and persistent foaming in the liquid seal chamber 16.

Fig. 6 shows the manner of assembling the separate portions of the present drainage device. Firstly the liquid seal chamber 16 is seated in the neck portion 50 of the container 12, with elastomeric sealing ring 62 in place about its upper edge. Correct orientation is ensured by the lug 114 and the notch 116. Secondly, the baffle assembly 54 is positioned in the liquid seal chamber 16, correct orientation being ensured by lug 110 and notch 112. Finally, the cap 53 is screwed onto neck 52 to seal the periphery of lid 20 tightly against the ring 62. The cap 53 also bears on the upper edge of the neck 52 and if desired another sealing ring 120 can be provided in the cap. The inlet hose 22 is attached to the inlet fitting 18 and to a drainage tube from the patient.

All parts of the present device are preferably constructed of a transparent and relatively rigid plastic material such as transparent butadiene styrene copolymer, acetate butyrate styrene copolymer or a like substance. When constructed of a relatively rigid plastic material the volumes of the various chambers do not vary substantially even though the pressure inside may vary somewhat due to the patient's respiration or the effect of the suction source. If all the various walls are made of a transparent material it is possible for an attendant to see into tube 92 and inspect the body fluids and gases being drained from the body cavity.

Before use, the user will precharge sterile water or a saline solution into the liquid seal chamber 16 sufficient to cover the open lower end of inlet tube 92. The chamber may be charged through the inlet fitting 18 or, if desired, through a separate orifice which can be closed by an elastomeric plug. In the preferred embodiment 60 to 85 ml of liquid is required.

In use fluids pass from the patient through the inlet tube 92 into the liquid seal chamber 16. Liquids overflow through tube 72 into the main collection chamber 14. Gases also pass through tube 72 and thence through outlet 124 to atmosphere or to the suction source.

Foam is generally confined to the space between the outer wall of the baffle and the inner wall of the seal chamber. The walls 96 & 98 ensure that the view of inlet tube 92 is undestructed by foam; the rise & fall of liquid can thus be observed. Bubbling of gas through the liquid seal can be observed directly through the wall 56.

If the unit is knocked over or even upended, liquid in the water seal chamber cannot be lost since the only exit is through outlet tube 72 which will inevitably lie above the free surface of the liquid. Results of tests are given below.

It has been found that this thoracic drainage unit provides effective and relatively long term collection and measurement of fluid drained from a patient's pleural cavity and it does so with minimal risk to the patient; the fluid passages remain relatively free from blockage. The free flow of fluid and the liquid seal maintained thereby is further assured by the size and nature of the various passages and parts provided therefor. It can be seen from the drawings that the cut away at the lower end of inlet tube 92 is at least as large as its diameter and that the cut outs 102 in the baffle walls 84 and 86 as well as opening 94 at the top of the overflow tube 72 are all larger in size then the diameter of tube 92. This means that anything that can enter from the patient should encounter easier passage at it moves through the liquid seal chamber.

Furthermore, no tortuous or convoluted passages are present which might become blocked by such substances.

In tests conducted with the thoracic drainage unit described a positive liquid seal of at least 12.5mm depth was maintained when the unit was tipped forward whilst essentially no loss at all of the liquid seal occurred when the unit was tipped to either side or backwards. In the drainage unit a liquid seal 12.5mm deep was maintained even under tidalling of as much as 600 mm in the inlet tube 92 and connecting tube 22. In extended tests the liquid seal retained its integrity in all practical tests.

Under tests of simulated multi-hour drainage and simulated pneumothorax the present thoracic drainage unit performed in a fully satisfactory manner. Gravity drainage was maintained throughout a 72 hour test employing a total of 2500 ml. of sanguinous fluids starting with 500 ml of whole blood followed successively by 500 ml of 75% whole blood and 25% plasma, 500 ml of 50% whole blood and 50% plasma and 1000 ml of 25% whole blood and 75% plasma with a flow rate diminishing exponentially from 200 ml/hour to 2 ml/hour at 72 hours. Blood clots of up to 10mm by 20 mm in area and 3 mm thick were added at intervals during the test. The simmulated respiration volume of 6000ml/ minute for half of each 6 second cycle was maintained freely throughout the test. The liquid seal was continuously maintained for the entire period. The addition of blood clots had no apparent effect since no blockage occurred.

Similar excellent results were obtained in a simulated 48 hour mediastinal drainage test under -20cm water vacuum and no air flow, wherein 2500 ml of whole blood was employed at an initial flow rate of 400 ml/hour diminishing exponentially to 0.9 ml/hour. Blood clots of similar size as above described were added at intervals throughout the test with no apparent effect on fluid or air flow rates. The liquid seal was maintained throughout the test at the full 24 mm depth. At the end of the test a sudden pneumothorax was simulated by applying vacuum of from 0 to -10 cm water. Patient breathing simulated by a positive 5 cm water of pressure showed no deterioration in the full 6000 ml/minute of air flow obtained in the initial gravity drainage tests. No blockage or increased resistance to fluid flow occurred as a result of the addition of blood clots.

**Claims**

1. A chest drainage device having a fluid collection chamber (14), and a closed transparent liquid seal chamber (16), including an overflow outlet (72) in fluid flow connection with said container (14), characterised thereby that said device includes a removable assembly (54) removably fixed and sealed within said chamber (16), said removable assembly including an inlet (18), a transparent inlet tube (92) spaced from a side wall (56) of said chamber (16) and in fluid flow connection with said inlet (18), said inlet tube (92) forming a lower edge opening (134) therein which is located within said chamber (16) between an overflow opening (96) of said overflow tube (72) and a bottom wall (58) of said chamber (16), and a baffle wall (96, 98) extending from said inlet tube (92) to said side walls (56), said baffle wall (96, 98) providing an enclosed space through which, in use, fluid in said inlet tube (92) may be viewed.

2. A device according to Claim 1 characterised thereby that said seal chamber (16) includes a further baffle wall (84-90) extending from the inlet tube (92) to said inner side to define an inner chamber around said overflow tube (72), said further wall (84-90) extending below the level of said overflow tube (72) and having one or more apertures (106) above said level.

3. A device according to Claim 2 characterised thereby that said further wall (84-90) is provided with cut outs (102) at the base adjacent said inner side.

4. A device according to Claim 2 or Claim 3 characterised thereby that said further wall (84-90) is 'U' shaped in plan, the inlet tube (92) being at the base of the 'U' and cut outs (102) being provided on both limbs (84,86).

5. A device according to Claim 4 characterised in that said apertures (102) are provided on both limbs (84,86).

6. A device according to Claim 5 characterised thereby that one round aperture(102) is provided on each limb(84,86).

7. A device according to any of Claims 2-7 characterised thereby that each aperture(102) is provided with a baffle(108) extending between said inner chamber and said inner side and extending around one side and below the respective aperture(102).

8. A device according to any preceding Claim characterised thereby that the seal chamber(16) is closed by a removable cap(20) to which said inlet tube(92) & baffle walls(84-90,96,98) are attached.

9. A device according to Claim 8 characterised thereby that an elastomeric seal(62) is provided between said cap(20) and the wall of said seal chamber(16).

10. A device according to Claim 8 or Claim 9 characterised thereby that the cap(20) is orientated in the seal chamber by location means(110,112).

11. A device according to Claim 10 characterised thereby that said seal chamber(16) includes graduations(132) thereon to indicate the fluid level in said inlet tube(92).

12. A device according to any preceding Claim characterised thereby that said seal chamber(16) is located in an upstanding tubular portion(50) of said container and is removeably secured therein by a threaded ring(53).

13. A device according to Claim 12 characterised thereby that the seal chamber(16) is orientated in said tubular portion(50) by location means.(114,116).

## Patentansprüche

1. Brustdrainagegerät mit einer Fluidsammelkammer (14) und einer geschlossenen, transparenten Flüssigkeitsverschlußkammer (16), mit einem Überlaufauslaß (72) in Fluidströmungsverbindung mit dem Behälter (14), dadurch gekennzeichnet, daß das Gerät eine abnehmbare Baueinheit (54) aufweist, die innerhalb der Kammer (16) abnehmbar befestigt und abgedichtet ist, und die einen Einlaß (18), ein transparentes Einlaßrohr (92) in Abstand von einer Seitenwand (56) der Kammer (16) sowie in Fluidströmungsverbindung mit dem Einlaß (18), wobei das Einlaßrohr (92) eine untere Randöffnung (134) darin bildet, die innerhalb der Kammer (16) zwischen einer Überlauföffnung (96) des Überlaufrohres (72) und einer Bodenwand (58) der Kammer (16) angeordnet ist, sowie eine Prallwand (96, 98) enthält, die sich vom Einlaßrohr (92) zu den Seitenwänden (56) erstreckt, und die einen umschlossenen Raum vorsieht, durch den beim Gebrauch Fluid im Einlaßrohr (92) betrachtet werden kann.

2. Gerät nach Anspruch 1, dadurch gekennzeichnet, daß die Verschlußkammer (16) eine weitere Prallwand (84-90) aufweist, die sich vom Einlaßrohr (92) zur Innenseite erstreckt, um eine innere Kammer rund um das Überlaufrohr (72) zu definieren, wobei sich die weitere Wand (84-90) unterhalb des Niveaus des Überlaufrohrs (72) erstreckt und eine oder mehrere Öffnungen (106) oberhalb dieses Niveaus hat.

3. Gerät nach Anspruch 2, dadurch gekennzeichnet, daß die weitere Wand (84-90) mit Ausschnitten (102) an der Basis benachbart der Innenseite versehen ist.

4. Gerät nach Anspruch 2 oder 3, dadurch gekennzeichnet, daß die weitere Wand (84-90) in Draufsicht "U"-förmig ist, wobei das Einlaßrohr (92) an der Basis des "U" ist und die Ausschnitte (102) an den beiden Schenkeln (84, 86) vorgesehen sind.

5. Gerät nach Anspruch 4, dadurch gekennzeichnet, daß dienungen (102) an beiden Schenkeln (84, 86) vorgesehen sind.

6. Gerät nach Anspruch 5, dadurch gekennzeichnet, daß eine runde Öffnung (102) an jedem Schenkel (84, 86) vorgesehen ist.

7. Gerät nach einem der Ansprüche 2 bis 7, dadurch gekennzeichnet, daß jede Öffnung (102) mit einer Leitfläche (108) versehen ist, die sich zwischen der inneren Kammer und der inneren Seite erstreckt, und die rund um eine Seite und unterhalb der jeweiligen Öffnung (102) verläuft.

8. Gerät nach einem der vorhergehenden Ansprüche, dadurch gekennzeichnet, daß die Verschlußkammer (16) durch eine abnehmbare Kappe (20) verschlossen ist, an der das Einlaßrohr (92) und die Prallwände (84-90, 96, 98) angebracht sind.

9. Gerät nach Anspruch 8, dadurch gekennzeichnet, daß eine elastomere Dichtung (62) zwischen der Kappe (20) und der Wand der Verschlußkammer (16) vorgesehen ist.

10. Gerät nach Anspruch 8 oder 9, dadurch gekennzeichnet, daß die Kappe (20) in der Verschlußkammer durch Lagemittel (110, 112) ausgerichtet ist.

11. Gerät nach Anspruch 10, dadurch gekennzeichnet, daß die Verschlußkammer (16) eine Skala (132) an ihr aufweist, um das Fluidniveau im Einlaßrohr (92) anzuzeigen.

12. Gerät nach einem der vorhergehenden Ansprüche, dadurch gekennzeichnet, daß die Verschlußkammer (16) in einem hochragenden rohrförmigen Teil (50) des Behälters angeordnet und darin mit Hilfe eines Gewinderinges (53) abnehmbar gesichert ist.

13. Gerät nach Anspruch 12, dadurch gekennzeichnet, daß die Verschlußkammer (16) im rohrförmigen Teil (50) durch Lagemittel (114, 116) ausgerichtet ist.

## Revendications

1. Appareil de drainage thoracique comportant une chambre collectrice de liquide (14) et une chambre de garde hydraulique transparente close (16), comprenant une sortie de trop-plein (72) en communication d'écoulement de liquide avec le récipient (14), caractérisé en ce que l'appareil comprend un ensemble amovible (54) fixé de manière amovible et scellé de manière étanche dans la chambre (16), cet ensemble amovible comprenant une entrée (18), un tube d'admission transparent (92) espacé d'une paroi latérale (56) de la chambre (16) et en communication d'écoulement de liquide avec l'entrée (18), le tube d'admission (92) formant une ouverture de bord inférieur (134) qui est placée dans la chambre (16) entre une ouverture de trop-plein (96) du tube de trop-plein (72) et une paroi de fond (58) de la chambre (16), et une paroi de chicane (96, 98) allant du tube d'admission (92) aux parois latérales (56), la paroi de chicane (96, 98) formant un espace clos à travers lequel, en service, le liquide contenu dans le tube d'admission (92) peut être observé.

2. Appareil suivant la revendication 1, caractérisé en ce que la chambre de garde (16) comprend une autre paroi de chicane (84-90) allant du tube d'admission (92) au côté intérieur pour définir une chambre intérieure autour du tube de trop-plein (72), cette autre paroi (84-90) s'étendant en dessous du niveau du tube de trop-plein (72) et comportant une ou plusieurs ouvertures (106) au-dessus de ce niveau.

3. Appareil suivant la revendication 2, caractérisé en ce que l'autre paroi (84-90) est pourvue de découpes (102) à la base adjacente au côté intérieur.

4. Appareil suivant la revendication 2 ou 3, caractérisé en ce que l'autre paroi (84-90) a la forme d'un U en plan, le tube d'admission (92) étant à la base du U et des découpes (102) étant prévues dans les deux ailes (84, 86).

5. Appareil suivant la revendication 4, caractérisé en ce que les ouvertures (102) sont prévues dans les deux ailes (84, 86).

6. Appareil suivant la revendication 5, caractérisé en ce qu'une ouverture ronde (102) est prévue dans chaque aile (84, 86).

7. Appareil suivant l'une quelconque des revendications 2 à 6, caractérisé en ce que chaque ouverture (102) est pourvue d'une chicane (108) qui s'étend entre la chambre intérieure et le côté intérieur et qui s'étend autour d'un côté et en dessous de l'ouverture (102) respective.

8. Appareil suivant l'une quelconque des revendications précédentes, caractérisé en ce que la chambre de garde (16) est fermée par un couvercle amovible (20) auquel le tube d'admission (92) et des parois de chicane (84-90, 96, 98) sont attachés.

9. Appareil suivant la revendication 8, caractérisé en ce qu'un joint d'étanchéité élastomère (62) est prévu entre le couvercle (20) et la paroi de la chambre de garde (16).

10. Appareil suivant la revendication 8 ou 9, caractérisé en ce que le couvercle (20) est orienté dans la chambre de garde par des moyens de positionnement (110, 112).

11. Appareil suivant la revendication 10, caractérisé en ce que la chambre de garde (16) porte des graduations (132) indiquant le niveau du liquide dans le tube d'admission (92).

12. Appareil suivant l'une quelconque des revendications précédentes, caractérisé en ce que la chambre de garde (16) est placée dans une partie tubulaire verticale (50) du récipient et y est fixée de manière amovible par une couronne à visser (53).

13. Appareil suivant la revendication 12, caractérisé en ce que la chambre de garde (16) est orientée dans la partie tubulaire (50) par des moyens de positionnement (114, 116).

Fig. 1

Fig. 2A

Fig. 2 B

Fig. 3

Fig.2

Fig. 4

Fig. 5

11

53

120

110

20

96

98

54

92

114

62

16

56

72

132

58

116

52

12

50

Fig. 6

12